(19) **Europäisches Patentamt / European Patent Office / Office européen des brevets**

(11) **EP 2 229 978 A1**

(12) # EUROPEAN PATENT APPLICATION

(43) Date of publication:
**22.09.2010 Bulletin 2010/38**

(51) Int Cl.:
**A61N 1/39** (2006.01)

(21) Application number: **10154339.5**

(22) Date of filing: **23.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **25.02.2009 AR P090100645**

(71) Applicants:
- **Carranza, Gustavo Ernesto**
  **Córdoba - AR (AR)**
- **Andrade, Marcelo Gabriel**
  **Buenos Aires - AR (AR)**

- **Andrade, Eduardo Javier**
  **Buenos Aires - AR (AR)**

(72) Inventors:
- **Carranza, Gustavo Ernesto**
  **Córdoba - AR (AR)**
- **Andrade, Marcelo Gabriel**
  **Buenos Aires - AR (AR)**
- **Andrade, Eduardo Javier**
  **Buenos Aires - AR (AR)**

(74) Representative: **Robba, Pierpaolo**
**Interpatent S.R.L.**
**Via Caboto 35**
**10129 Torino (IT)**

(54) **A control system to generate defibrillation waves of automatically compensated charge without measurement of the patient impedance**

(57)    The present invention discloses a system to control the generation of biphasic defibrillation waves and its main feature is to eliminate the need of measuring the patient impedance. This simplification is achieved by delivering the charge in pulses and making this charge proportional to the instant charge remaining in the storage capacitor at the end of each pulse or sample cycle. To this end, the charge delivered to the patient (integral of the current) is compared with a fraction of the voltage present in the capacitor, annulling the current in the instant that the integral of the current reaches the value of said fraction. In this way, the charge in the positive phase and the charge in the negative phase are exclusively a function of the selected dose (energy or charge, as preferred) and constant for different values of patient impedance, and the necessary voltage in the capacitor is exclusively a function of the selected dose.

FIGURE 4

EP 2 229 978 A1

**Description**

*Technical Field of the Invention*

The present invention refers to a control system to generate defibrillation waves, of automatically compensated charge, that does not need to measure the patient impedance. In particular, the present invention refers to a control system to generate waves of the kind used for the biphasic defibrillation.

*Background of the Invention*

Throughout this specification reference is made to several publications, including patent publications, which are cited in brackets as they appear in the "List of References" appended hereto; all of these publications are incorporated herein by reference in their entirety.

Both the defibrillation and electric cardioversion consist of types of therapy that, through the application of a direct current electric shock, revert different mortal disorders of the cardiac rhythm. Its high efficacy, application simplicity and security have contributed to its great diffusion, being available in almost any area of health assistance, and even the automatic ones in public places, without any health personnel.

Defibrillation is used in cases of a cardiorespiratory arrest, with an unconscious patient, that present ventricular fibrillation or ventricular tachycardia without pulse. Without treatment they are lethal.

Electric cardioversion is used to revert any kind of reentrant arrhythmias except for ventricular fibrillation. The electric shock is synchronized with the electric activity of the heart. It can be administered in a selective or urgent way, if the situation compromises the life of the patient.

Claude Beck performed the first defibrillation during a heart surgery in 1947.

The cardioversion was first used in humans by Zoll et al. during the 50's to treat the atria fibrillation through alternating current shocks that frequently induced ventricular fibrillation. Shortly afterwards, Lown et al. drastically reduce this complication by performing the same with direct current. Later, this would disappear by introducing the synchronization with the R wave of the electrocardiography (ECG), i.e. to transmit the discharge with the depolarization of the ventricles avoiding executing it during the ventricular re-polarization, the T wave of the electrocardiography.

The direct current shock that continues on the heart induces the simultaneous depolarization of all the myocardial cells, which cause a pause for re-polarization; and afterwards, if they have succeeded, the heart takes up again its normal electric rhythm, with depolarization and muscular contraction, first the atria and then the ventricles. The success of the treatment depends both in the underlying pathology as well as the density of the current that reaches the myocardium and its duration.

**External Defibrillator**

The energy is administered through some paddles or electrodes placed in the thorax, in the cutaneous surface.

**Manual or Conventional Cardioverter-Defibrillator**

Is the one used by medical staffs. There is a screen showing a portion of the electrocardiography and the doctor decides the intensity and if he synchronizes the discharge with the R wave. If there is no synchronization, it would be a defibrillation and, if there is synchronization, it would be a cardioversion.

**Automatic External Defibrillator**

The Automatic External Defibrillator (AED) may be semi-automatic if the apparatus detects the arrhythmia and notifies the operator to release the energy or completely automatic if the intervention of an operator is not required to release the energy.

**Internal Defibrillator**

The energy is administered in the endocardium through wires-electrodes. Much less energy is needed. This technique uses the Automatic Defibrillator Implantable (ADI) in which the generator is implanted in the subcutaneous tissue with wires-electrodes generally located in the right cardiac cavities. The current models are of biphasic waves. They are placed on patients with risk of a ventricle fibrillation.

**Monophasic**

These ones were used until this date, and, even though they are currently the most used model, they tend to disappear. They discharge unipolar current, i.e. a unique way of current flow. The commonly employed dose is a defibrillation with this apparatus of up to 360 Joules.
Within this group there are two kinds of waves, the damped sine monophasic, in which the current flow gradually goes back to zero, and the truncated exponential monophasic, in which it is electronically terminated before the current flow reaches to zero.

**Biphasic**

They discharge current that flows in a positive direction during a certain amount of time before reverting and flowing in a negative direction during the last milliseconds of the discharge. They are more efficient, taking only approximately half the energy of the monophasics. In the front of the apparatus it should appear the range of the effective dose. If it is unknown, 200 Joules shall be used. Generally, 2 to 4 Joules/Kg are used for an adult in the case of defibrillation, and from 0.5 to 1 J/Kg in the case of cardioversion.
This group has three main wave forms, truncated exponential biphasic, rectilinear biphasic and sampled exponential biphasic.
Currently, the defibrillators of monophasic current are being replaced by the ones with biphasic current, which are more convenient since it has been proved, throughout the years that, the necessary energy to obtain the same therapeutic effects is lower, considerably decreasing the unwanted side effects such as skin burns, myocardium tissue damage, refibrillation, etcetera. (14), (15), (16), (18), (26), (63) and (82)
The use of a biphasic defibrillation wave implies the delivery of current to the patient in two phases, the first with direct current and the second with an inverted current. (1), (2), (4) to (7), (10), (11), (12), (14), (19), (32) and (34)
The state of the art in the biphasic defibrillation quantifies the dose administered to the patient in energy delivered on determined patient impedance. (13), (31), (32) and (64) The current is administered through a capacitor (or more capacitors), which has been previously charged with a tension value, based in the energy dose to deliver. (1)
The most widespread form of this technique is the one called "Truncated Exponential Biphasic", which consists in directly discharging a capacitor in the patient, reversing the direction of the current in a fraction of the whole time and finishing the discharge prior to the annulment of the tension in the capacitor. (1), (20), (21), (24), (25) and (27)
There is a general scientific consent regarding the fact that the improvement in the efficacy of this method is due to the membrane potentials restoration of the myocardium tissue produced by the second phase, in which the direction of the current is reversed. (18) and (26)
The usual discharge times in most of the manufacturers ranges between at least 7 ms and at the most 20 ms. The current circulation is interrupted before the same goes down to zero. There are no unified criteria regarding the optimal duration of the discharge. (9), (18), (52), (57), (59), (60), (61), (62), (65), (66) and (82)
The "Tilt Factor" of the wave is defined as the difference between the initial current minus the final divided by the initial value. (3)

$$K_D = \frac{I_0 - I_F}{I_0} \quad (1)$$

where $I_0$ is the current in the initial moment and $I_F$ is the current in the final moment of the discharge.
Being an exponential wave of T seconds, it may be calculated as

$$K_D = \frac{I_0 - I_0 e^{-T_T/\tau}}{I_0} = 1 - e^{-T_T/\tau} \quad (2)$$

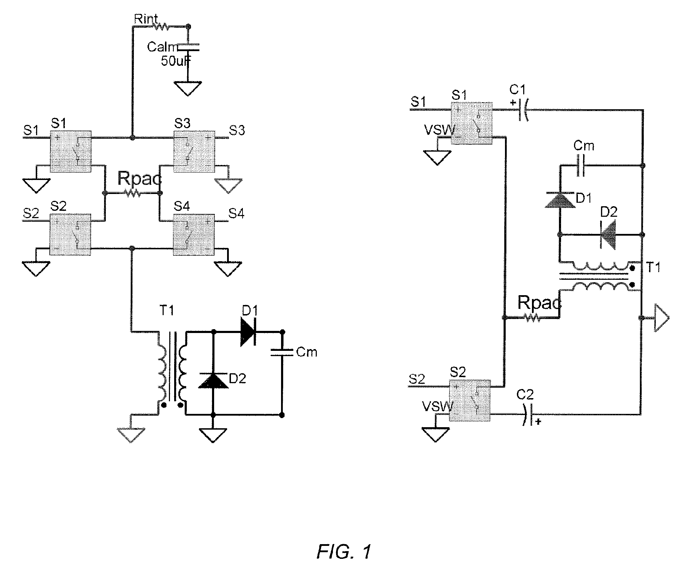

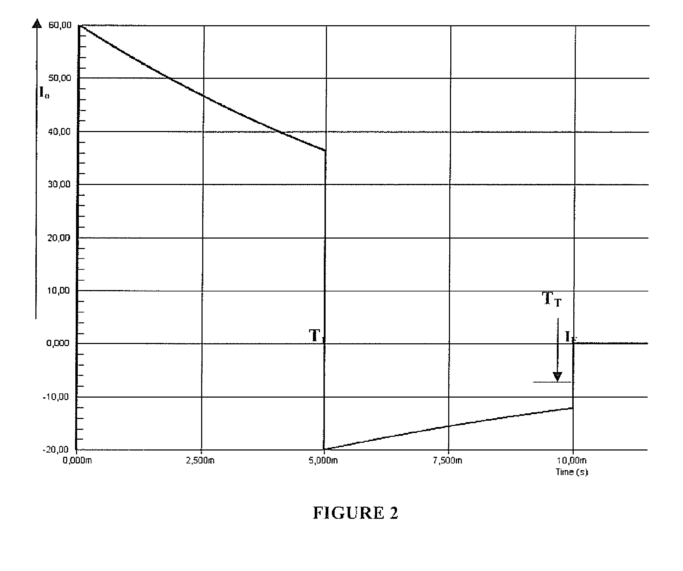

where $T_T$ is the complete duration of the pulse and $\tau$ the time constant belonging to the equivalent RC circuit. (See Figure 2)
The application of this form of energy delivery is executed through what is known as "H-bridge".
Referring to Fig. 1, on the left it is shown the truncated exponential biphasic discharge implementation diagram of the circuit known as the "H-bridge": while switches 1 and 4 are closed, the current circulates from left to right, then, these would open and S2 and S3 are closed, producing the inverse current; it may be replaced by a two capacitor-two switch system as shown on the right.

A current minimum threshold value during a minimum time is necessary for the defibrillation to act and from determined values and onwards, there is a risk of tissue damage. (1), (8), (10) and (13)

It is necessary to take into account the patient impedance in each discharge, in order to avoid that the current values are excessively high for patients with low impedance or not enough for patients with high impedance, since impedance among different patients or the same patient in different conditions may vary in a relation of 7 to 1. (28), (29), (30), (75), (82) and (83)

The patient impedance is defined as the relation among the tension applied to the paddles on the thorax and the current flowing through the same. Since a determined tension, i.e. the one stored in the capacitor, is available to be applied on the paddles, if the necessary precautions are not taken, the current that will be effectively applied on the same is of a great uncertainty.

It has been proved that the patient impedance:

- is not purely resistive, (83)
- varies in time during the discharge, (69)
- is current dependant. (68)

If the patient impedance changes, the relationship between the delivered charge in each phase and the maximum and minimum current values in each phase changes, thus the desired restoration on the membrane potentials may not occur or the currents may result harmful for the patient.

All the implemented solutions until this moment for this inconvenience imply the measurement of the patient impedance and the election of a method to compensate the unwanted effects of this dispersion. These measurements may be achieved by incorporating measuring electronic devices, which consist of a hardware and sophisticated software. (28), (29), (30), (35), (36), (37), (38), (40), (45), (46), (50), (51), (58), (67) to (75)

Until this moment, solutions that imply eliminating the need of measuring the patient impedance, and consequently, avoiding the uncertainty that those measurement methods carry in the effectively administered charge values and reducing the risk of possible failures have not been proposed.

*Disclosure of the Invention*

The present document discloses a control system to generate truncated exponential biphasic defibrillation waves with compensated automatic charge that eliminates the need to measure the patient impedance.

The system proposed in this application, contrary to the ones described in the state of the art, by accomplishing an automatic and instantaneous compensation of the possible variations of the patient impedance, make unnecessary to measure it, allowing the elimination of the electronic measuring devices.

Thus, the method of charge provision is simplified, avoiding one step, minimizing the risks and reducing costs.

As an extra benefit, the device works as both a biphasic as well as a monophasic defibrillator, allowing the update of the latter to work as biphasic, only by replacing the discharge module for one designed according to our proposal.

The features of the system disclosed in this presentation are both innovative and superior to the previous ones available in the market, as it has been detailed above.

For a better understanding of the present invention, and in order to easily put the same into practice, a precise description of a preferred embodiment will be given in the following paragraphs referring in the same to the accompanying illustrative drawings, the whole with exclusive exemplary character purely demonstrative, but not limitative of the invention, components of which may be selected among different equivalents without departing from the scope of the invention set forth in this document.

*Brief Description of the Drawings*

The following drawings are attached, which graphically describe the operation of the system described.

Fig. 1 shows, on the left, a truncated exponential biphasic discharge implementation diagram of the circuit known as the "H-bridge" while, on the right, it is replaced by a two capacitor-two switch system.

Fig. 2 graphically represents an example of a conventional truncated exponential biphasic discharge.

Fig. 3 represents two diagrams that exemplifies a charge delivery controlling the wide of the pulse (current and charge delivered based on time) of a "Class D Amplifier" applied to a biphasic discharge.

Fig. 4 represents a possible practical implementation for the wave form 3.

Fig. 5 shows the replacement of the resistive voltage divider R1//R2 of Fig. 4 by a voltage signal variable through time.

*Description of a Preferred Embodiment*

As aforesaid, the present document reveals a control system to generate truncated exponential biphasic defibrillation waves with compensated automatic charge that eliminates the need to measure the patient impedance.

The application of waves as the ones described in the biphasic defibrillation, whether it is external or internal, allows, thanks to the great simplicity and versatility of the wave conformation, to achieve higher results in the defibrillation process regarding previous devices and systems, essentially due to the following:

1. it eliminates the need of measuring the patient impedance;
2. it delivers the electric charge through a fixed amount of charge packages, where each of them is proportionate to the remaining charge in the energy capacitor;
3. it allows the establishment of a method to adjust, pulse by pulse, the delivered charge fraction;
4. the total electric charge delivered to the patient is compensated; and
5. the result is a technical solution simpler and safer than those currently available to implement this method.

The invention is based in the hypothesis that the therapeutic action of the defibrillator revolves around the current delivered to the patient and the time of delivery, more than in the energy.

Therefore, its objective is to maintain the total charge delivered to the patient constant and to fix the relation between charges in each phase.

In this sense, the charge delivered in each phase and the total charge are functions of the selected dose, either energy or charge as it may be desired, and to remain constant for different values of patient impedance.

This can be achieved by delivering to the patient a fixed amount of charge in the straight direction and another, lower, in the inverse direction, in a proportion that is adjustable according to the physiological requirements to restore to zero the membrane potentials in the myocardium cells.

$$Q_{TE} = \int_0^{T_T} |i|\,dt = cte$$

$$Q_{F1} = \int_0^{T_1} |i|\,dt = A \times Q_{F2} = A \times \int_{T_1}^{T_T} |i|\,dt \quad A>1$$

where:

$Q_{TE}$   Total charge delivered to the patient
$i$   Instantaneous electric current
$Q_{F1}$   Total charge delivered in straight direction
$Q_{F2}$   Total charge delivered in inverse direction
$T_1$   Duration of the straight phase
$T_T$   Total duration of the discharge
$A$   Relation between the straight and inverse charges

The method to evaluate this values and requirements is already known. (35) and (53) to (56)

The charge is delivered in pulses, which wide is independently adjusted, so as the charge in each phase and the total delivered are independent from the patient impedance, within the impedance range of interest.

"Packages" of charge proportional to the total charge present in the capacitor of each sample cycle are provided.

These pulses with a controlled wide are of a much higher frequency that the inverse of the total time of energy delivery. A typical value may be $T_T$ = 10 ms, which implies a sample frequency of, at least $F_R$ = 10/$T_T$ = 1 kHz. This system is known as "Class D Amplifier" and is used in diverse applications in the electronic industry. (17), (33), (39), (42), (76) and (84).

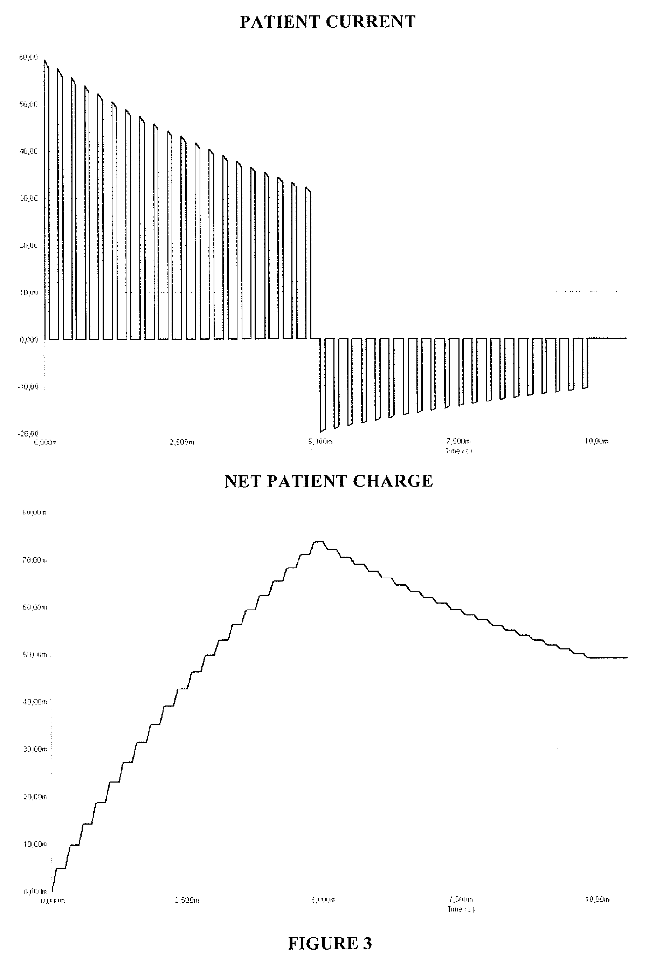

Referring to Fig. 3, which is a graphical representation of the "Class D Amplifier" applied to the biphasic discharge, two diagrams that exemplify a charge delivery controlling the wide of the pulse (current and charge delivered based on time) are shown.

All prior systems need to measure the patient impedance and calculate the parameters of the discharge from the same. The proposed system, on the contrary, by measuring and controlling the delivered charge, does not need to measure the impedance nor compensate to control the delivered charge. (35) to (51)

The total charge provided to the patient ($Q_{TP}$), adding absolute direct current values plus inverse, is:

$$Q_{TP} = \sum_{i=1}^{i=n} Q_i \quad (3)$$

$Q_i$ : amount of charge provided to the patient in the cycle "i", n= $T_T/T_c$ (total time over time of sample cycle)

The charge delivered to the patient is identical to the variation of charge stored in the $Q_E$ capacitor

$$Q_{TP} = Q_E = (V_0 - V_R)C = V_0\left(1 - e^{-T_1/\tau}\right)C = CV_0 K_D \quad (4)$$

Basically, it is a RC circuit with an initial charge $Q_{C0}$ that responds to the differential equation:

$$V = R \times I \Rightarrow \frac{Q_C(t)}{C} = R\frac{dQ_C}{dt}$$

where

$$dQ_C = \frac{Q_C(t)}{RC}dt \quad (5)$$

Transforming the differential equation to its approximation by finite differences:

$$Q_{C(i-1)} - Q_{Ci} = \frac{Q_{C0} - \sum_{j=1}^{j=i} Q_j}{RC}\left(t_i - t_{i-1}\right) \quad (6)$$

where we make constant $(t_i - t_{i-1}) = T_C$
and where we name $Q_i$ to the charge delivered in pulse "i" of the discharge

$$Q_{C(i-1)} - Q_{Ci} = Q_i \quad (7)$$

(the charge delivered to the patient is equal to the variation of the charge in the capacitor)

We propose that the delivered charge in each sample cycle $Q_i$ is proportional, in a small fraction $\alpha$ much lower than 1, to the remaining charge in the capacitor $Q_{Ci}$ ($\alpha \ll 1$)

$$Q_i = \alpha Q_{Ci} = \int_0^{T_C} i\,dt \quad (8)$$

Solving the integral for cycle "i"

$$Q_i = \alpha Q_{Ci} = Q_{Ci-1} - Q_{Ci} = Q_{Ci-1}\left(1 - e^{-T_C/\tau}\right) \quad (9)$$

where $\tau = (R_{pac}+R_{int})C$

$$Q_i = \alpha\left(Q_{Ci-1} - Q_i\right) \Rightarrow Q_i = \frac{\alpha}{\alpha+1} Q_{Ci-1} \quad (10)$$

In comparison with (9) results

$$\frac{\alpha}{\alpha+1} = \left(1 - e^{-T_C/\tau}\right) \quad (11)$$

To technically implement this proposal, the charge is delivered connecting during a fraction of the sample cycle the charge to the storage capacitor. We name dutty cycle $D_i$ to the relation between the necessary time to deliver charge $Q_i$ and the duration of the sample cycle

$$D_i = \frac{T_{ON}}{T_C} \quad (12)$$

Since $T_C$ is fixed and $T_{ON} \le T_C$, then, for each R value there exists a maximum value C that makes possible to reach the delivery of the desired complete charge in that maximum time. Setting the maximum resistance value and the C value, the a value is determined.

Also, it has to be taken into account the internal resistance $R_{int}$ of the discharge circuit to determine the $\alpha$ value.

The initial voltage of the capacitor and the total charge delivered are defined from the delivery of a defined energy value for a normalized patient's resistance of, for example, 50 Ohm. In this way, the charge doses that we propose are correlated with the energy that is the current convention.

If the internal resistance of the discharge circuit is null, then the relation between the delivered energy and the charge is constant

$$E_E = \frac{C\left(V^2_0 - V^2_R\right)}{2}\eta = \frac{C\left(V^2_0 - V^2_R\right)}{2}\frac{R_{pac}}{R_{pac} + R_{int}} = \frac{CV^2_0\left(1 - (1 - K_D)^2\right)}{2}\frac{R_{pac}}{R_{pac} + R_{int}} \quad (13)$$

Since we maintain the total charge delivered constant, the delivered energy results function of the patient impedance and the internal impedance. This imposes restrictions to the internal resistance of the discharge circuit if we want to maintain the energy variation delimited.

Defining the maximum tilt factor we may calculate the necessary minimum capacity value

$$C_{min} = \frac{-T_T}{R_{Max}L_N(1 - K_D)} \quad (14)$$

Given $R_{Max}$, $T_C$ and selecting C, with equation (11) we calculate the $\alpha$ value, which defines the proportion of charge delivered to the patient to charge remaining in the capacitor. Afterwards, we may be able to calculate the wide of the pulse necessary to accomplish the charge condition delivered per cycle through the integral equation.

Considering that the integral between 0 and $T_C$ of the equation (8) results equal to the integral between 0 and $T_{ON}$ by annulling in that instant the current,

$$T_{ON} = -\left(R_{pac} + R_{int}\right) \times C \times Ln\left(\frac{\alpha}{1-\alpha}\right) \quad (15)$$

It can be seen that the relation between patient-impedance and useful cycle is linear. Since we can measure the delivered charge and compare it with the remaining in the capacitor, this calculation is unnecessary, the current delivery is simply inhibited in each cycle when reaching the desired charge value.
With this system, the following is avoided:

- to measure the patient impedance,
- to calculate the wide of the pulse,
- to evaluate possible modifications of the impedance in time, and
- to evaluate possible modifications of the impedance based in voltage.

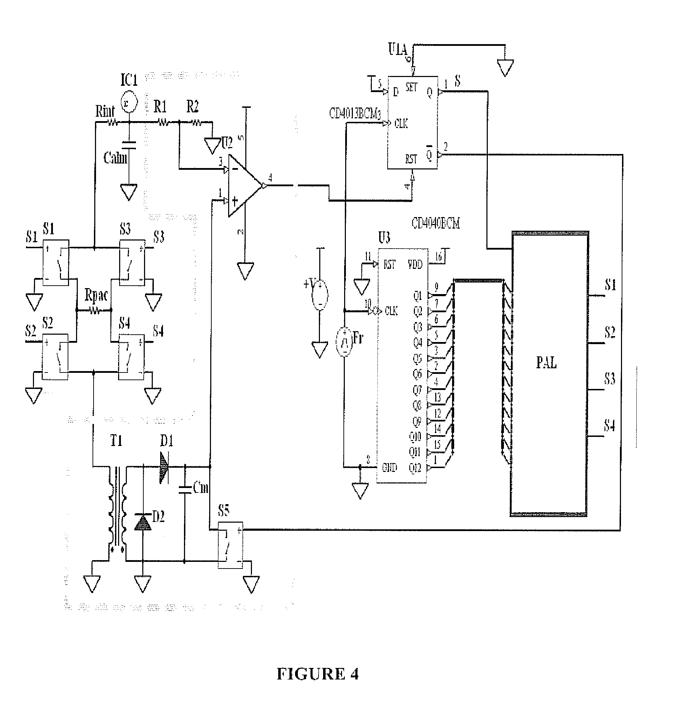

The implementation is reduced to a minimum increasing the confidence and efficiency of the discharge module by eliminating the complexity of impedance measuring devices. Once the energy to deliver, capacity, circuit maximum resistance and total time values are defined, the initial voltage value of the capacitor is determined from them.
The charge value is determined from the reference energy delivered to a resistance typical nominal value, for example 50 Ohm, through the equation (13).
The technique to implement this method consists in controlling the wide of each pulse so as the delivered charge is the programmed one. This is achieved through a "H-bridge" that initiates each cycle connecting the patient to the adequate direction, measuring delivered current and integrating the same on a capacitor until the charge is the desired one.
All prior systems need to measure the patient impedance and calculate the wide of the pulse from the same.
On the contrary, the proposed system, when measuring the delivered charge, does not need to measure the impedance nor compensate to control the delivered charge. The impedance variations are compensated in an automatic and instantaneous way.
To clear out the ideas, and making reference to Fig. 4, we present an example and the implementation method:
Fig. 4 represents a possible practical implementation for the wave form 3; the circuit that allows to integrate delivered charge in each pulse (lower zone) and to compare it with a charge fraction present in the capacitor ($R_1$ and $R_2$ voltage divider) is highlighted in broken line: it is the key point that makes unnecessary the measurement of the patient impedance.
On the right of Fig. 4 there is a block diagram of a simple logic implementation to control the wave form: the switches S1 and S4, commuting at the frequency of reference, they control the wide of the pulse during first phase and switches S2 and S3 during second phase; each pulse begins with the positive flank of the clock signal and ends when the tension in the $C_m$ capacitor reaches the value $V_{Calm}*R_2/(R_1+R_2)$.
We assume that:

| | |
|---|---|
| Delivered energy: | 200 Joules |
| Reference impedance: | 50 $\Omega$ |
| Sample Frequency: | 5 kHz |
| Total Time: | 10 ms |
| Tilt Factor: | KD $\leq$ 0.65 |
| Maximum Patient-Impedance: | $R_{Pmax}$ = 200 $\Omega$ |
| Internal Resistance: | $R_{Int}$ =5 $\Omega$ |

From (13): $C \geq$ 46.5 uF
We select C=50 uF
From (2):

$$K_D = 0.6230$$

From (14), for 50 $\Omega$:

$$V_0 = \sqrt{\frac{2E_E\left(R_{pac} + R_{int}\right)}{R_{pac}C\left(1 - (1 - K_D)^2\right)}} \approx 3{,}203\,\text{V}$$

From (4):

Delivered charge:   $Q_{TP}$ = 99.8 mC
Initial charge:      $Q_0 = V_0 * C$ =160.1 mC

From (11):

Discharge coefficient   $\alpha$ = 0.019704

The functioning begins, after reaching the corresponding voltage in the capacitor $C_{alm}$ and given the discharge order, starting the oscillator Fr. Then, the control logic is in charge of closing the S1 and S4 switches. Simultaneously, the S5 switch is opened, allowing the beginning of the integration of the current delivered to the patient until the moment in which the tension in $C_m$ is leveled in the fraction corresponding to the tension in $C_{alm}$.

In this moment, it discharges $C_{alm}$ and the switches are opened until the beginning of a new pulse of Fr. When reaching time $T_1$ corresponding to the inversion, the S 1 and S4 switches stop being activated and S3 and S4 are activated. When completing the discharge time $T_2$ the S1 to S4 switches are opened again and S5 is closed.

Adding up the tension $V_m$ values obtained in each cycle, or performing an independent integration, it can be verified if the total charge effectively delivered to the patient is the desired one.

The comparison in U2 implies   $V_{Cm} = \dfrac{Q_i}{nC_m} = \dfrac{Q_{Ci}}{C_{alm}} \times \dfrac{R_2}{R_1 + R_2}$   where the condition design for the divisor and the integrator with current transformation design arises, for instance (analogical or digital active integrated circuits may be used also)   $\alpha = \dfrac{nC_m}{C_{alm}} \times \dfrac{R_2}{R_1 + R_2}$   n is the relation of windings of the current transformer $T_1$

We set $(R_1+R_2)C_{alm}$ >> T internal discharge (30 seconds, for example)

$$C_m = \frac{Q_1}{nV_{Cm}} = \frac{0.00318599}{100\text{x}4\text{V}} \approx 7.964975$$

Fixing n and $C_m$ to obtain a maximum comparison tension, for maximum energy, of around 4 V we may calculate the divisor.   $C_m = \dfrac{Q_1}{nV_{Cm}} = \dfrac{0.00318599}{100\text{x}4\text{V}} \approx 7.964975$   We selection C = 10 uF so that $V_{Cm}$ = 3.186

The divisor will compensate by adjusting the dispersions in the capacities values.

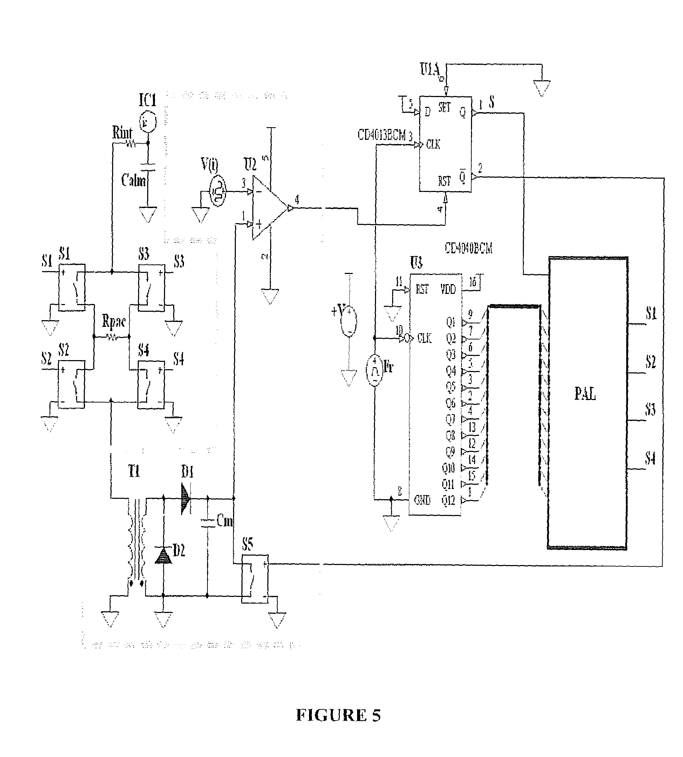

The rest of the circuit may be designed according to the usual procedures of the electronic. On the other hand, if the resistive divisor is replaced by a controlled voltage (see Fig. 5), the amount of delivered charge is programmed pulse by pulse, while the resultant wide of the pulse does not reach the sample time.

In this way, the charge / time distribution during the discharge may be completely configured within this limit.

As a matter of facts, Fig. 5 shows how, through the replacement of the resistive voltage divider R1//R2 in Fig. 4 scheme, by a voltage signal variable through time, the delivered charge in each pulse may be voluntarily determined, rendering infinite temporal charge distributions based in time.

In this specification, we have shown that the proposed method allows, in a very simple form, to implement a discharge wave generation system for a high reliability biphasic defibrillator that allows dispensing from the impedance measuring devices, simplifying considerably the traditional operation and eliminating any consequence derived from eventual measuring errors.

"A control system to generate defibrillation waves of automatically compensated charge without measurement of the patient impedance"

LIST OF REFERENCES

1. Tacker, W. A. "External Defibrillators." The Biomedical Engineering Handbook: Second Edition. Ed. Joseph D. Bronzino Boca Raton: CRC Press LLC, 2000

2. "Cardioversión Bifásica vs Monofásica en una serie consecutiva. ¿Qué aportan de mejora los nuevos desfibriladores bifásicos frente a los convencionales?" (Biphasic vs Monophasic Cardioversion in a consecutive series. What is the contribution of the new biphasic defibrillators compared to the conventional ones?) Marin I., Hernández Madrid A., Gómez Bueno M., Escobar Cervantes C., Moro Serrano C. Ramón y Cajal Hospital, Madrid, Spain. "4to. Congreso Virtual de Cardiología" (4th Cardiology Virtual Congress)

3. Intracardiac atrial defibrillation
Derek J. Dosdall, Ph.D.1 and Raymond E. Ideker, M.D, Ph.D.1,2,3 Heart Rhythm. 2007 March; 4(3 Suppl): S51-856.
www.pubmedcentral.nih.gov/articlerender.fcgi?artid=1868675

4. Biphasic versus Monophasic Shock for External Cardioversion of Atrial Flutter. A Prospective, Randomized Trial
Kai Mortensen1, Tim Risiusl, Tjark F. Schwemer, Muhammet Ali Aydin, Ralf Köster, Hanno U. Klemm, Boris Lutomsky, Thomas Meinertz, Rodolfo Ventura, Stephan Willems www.content.kargel.com/ProdukteDB/produkte.asp? Aktion=ShowFulltext&Artikel Nr=000113429&Ausgabe=234531 &ProduktNr=223832

5. Comparative efficacy of monophasic and biphasic waveforms for transthoracic cardioversion of atrial fibrillation and atrial flutter Osnat T. Gurevitz, MDa, Naser M. Animash, MDa, Joseph F. Malouf, MDa, Krishnaswamy Chandrasekaran, MDa, Ana Gabriela Rosales, MSb, Karla V. Ballman, PhDb, Stephen C. Hammill, MDa, Roger D. White, MDc, Bernard J. Gersh, MB, ChBa, Paul A. Friedman, MDa Received 20 January 2004; accepted 4 July 2004.
http://www.ahjonline.com/article/S0002-8703(04)00417-X/abstract

6. Atrial fibrillation. Monophasic versus biphasic waveform shocks for atrial fibrillation cardioversion in patients with concomitant amiodarone therapy.
Vitor S. Kawabata, Caio B. Vianna*, Miguel A. Moretti, Maria M. Gonzalez, João F. Ferreira, Sergio Timerman and Luiz A. Cesar
Heart Institute (INCOR-HCFMUSP), University of São Paulo Medical School, Av. Doutor Enéas Carvalho Aguiar 44, 05403.000 São Paulo, Brazil
www.europace.oxfordjournals.org/cgi/content/abstract/9/2/143

7. Predictors of success and effect of biphasic energy on electrical cardioversion in patients with persistent atrial fibrillation.
Josep M. Alegret1,*, Xavier Viñolas2, Jaume Sagristá3, Antonio Hernandez-Madrid4, Luisa Pérez5, Xavier Sabaté6, Lluís Mont7, Alfonso Medina on behalf of the REVERSE Study Investigators8
Europace Advance Access originally published online on June 2, 2007 Europace 2007 9(10):942-946; doi: 10.1093/europace/eum107
www.europace.oxfordjournals.org/cgi/content/abstract/9/10/942

8. Recommendations 2005 of the European Resuscitation Council on Cardiopulmonary Resuscitation. Section 2. Basic vital support in adults and the use of external automatic defibrillators
Anthony J. Handley, Rudolph Koster, Koen Monsieurs, Gavin D. Perkins, Sian Davies, Leo Bossaert
https://www.erc.edu/index.php/guidelines download 2005/en/

9. Low-Energy Biphasic Waveform Defibrillation: Evidence-Based Review Applied to Emergency Cardiovascular Care Guidelines: A Statement for Healthcare Professionals From the American Heart Association Committee on Emergency Cardiovascular Care and the Subcommittees on Basic Life Support, Advanced Cardiac Life Support, and Pediatric Resuscitation.
Richard O. Cummins, Mary Fran Hazinski, Richard E. Kerber, Peter Kudenchuk, Lance Becker, Graham Nichol, Barbara Malanga, Tom P. Aufderheide, Edward M. Stapleton, Karl Kern, Joseph P. Omato, Arthur Sanders, Terence Valenzuela and Mickey Eisenberg Circulation 1998;97;1654-1557
www.circ.ahajournals.org/cgi/content/full/97/16/1654

10. The Role of Biphasic Shocks for Transthoracic Cardioversion of Atrial Fibrillation Simon J Walsh, MD MRCP; Ben M Glover, MB BCh MRCP; AA Jennifer Adgey,
MD
FRCP FACC Regional Medical Cardiology Centre, Royal Victoria Hospital, Belfast.
Indian Pacing and Electrophysiology Journal (ISSN 0972-6292), 5(4): 289-295 (2005)
www.pubmedcentral.nih.gov/articlerender.fcgi?artid=1431603

11. Biphasic Defibrillation: Increasing Efficacy While Decreasing Risk Clinical Considerations
Michael R. Gold. M.D., PhD. Chief, Division of Cardiology Medical Director Heart and Vascular Center Medical University of South Carolina. Charleston, SC Medtronic Corporation 2002

12. Conditioning prepulse of biphasic defibrillator waveforms enhances refractoriness to fibrillation wavefronts. JF Swartz, JL Jones, RE Jones and R Fletcher Circ. Res. 1991;68;438-449 American Heart Association. 7272 Greenville Avenue, Dallas, TX 72514

13. Energy Levels for Biphasic Defibrillation. An Advisory Statement from the Australian Resuscitation Council. Ian Jacobs; James Tibballs; Peter Morley; Jennifer Dennett; Jeff Wassertheil; Vic Callanan; John Hall: ARC executive committee on behalf of the Australian Resuscitation Council* June 2003

14. Demystifying biphasic defibrillation Elaine Amato-Vealey, RN, PhD Patricia A. Colonies, RN, CCRN, Med Nursing2005 August 2005 Volume 35 Number 8 - Supplement: ED Insider Pages 6 -11

15. Attenuating the defibrillation dosage decreases post resuscitation myocardial dysfunction in a swine model of pediatric ventricular fibrillation. Laboratory Investigations Pediatric Critical Care Medicine. 9(4):429-434, July 2008. Berg, Marc D. MD; Banville, Isabelle L. PhD; Chapman, Fred W. PhD; Waker, Robert G. BS; Gaballa, Mohammed A. PhD; Hilwig, Ronald W. PhD, DVM; Samson, Ricardo A. MD; Kern, Karl B. MD; Berg, Robert A. MD www.pccmjournal.com/pt/re/pccm/abstract.00130478-200807000-00014.htm;jsessionid=JBvL9MW2TL8LjFzQ1vzRHJcQZTD9RcXgnhg7hL8B2n4 1 vg14bLh6!136317464!181195628!8091!-1

16. Biphasic Transthoracic Defibrillation Causes Fewer ECG ST-Segment Changes After Shock Ramakota K Reddy MD, Marye J Gleva MD, Bradford E Gliner MSBME, G.Lee Dolak MD, Peter J Kudenchuk MD, Jeanne E Poole MD and Gust H Bardy MD

17. Experimental Evidence of Improved Transthoracic Defibrillation With Electroporation-Enhancing Pulses. Malkin, R.A. Guan, D. Wikswo, J.P. This paper appears in: Biomedical Engineering, IEEE Transactions on Publication Date: Oct. 2006 Volume: 53 , Issue: 10

18. MultipulseBiowave_EN_frei.PDF www.schiller.ch/ps/em/docudb/downloads/schiller2006_docudb/N2ZhZTBjZTA3Z GMyYWJmODEwNmFjMGEwYzBhYzOxYzgzYjR1M2M2OGIzNGFhZTg1NTE wOGM3Y2ZjODNmMzZIZS0tcHMxXy1kb2MtLQ=/MultipulseBiowave EN_fr ei.PDF

19. Defibrillation. Circulation 2005;112;III-17-III-24 DOI:0.1161/CIRCULATIONAHA.105,166473 American Heart Association

20. External cardioversion of atrial fibrillation: comparison of biphasic vs monophasic waveform shocks. P. Ricard1, S. Le'vyl, G. Boccaral, E. Lakhall and G. Bardy2 www.europace.oxfordjournals.org/cgi/reprint/3/2/96

21. Monophasic versus biphasic waveform shocks for atrial fibrillation cardioversion in patients with concomitant amiodarone therapy. Vitor S. Kawabata, Caio B. Vianna*, Miguel A. Moretti, Maria M. Gonzalez, Joa~o F. Ferreira, Sergio Timerman, and Luiz A. Cesar Heart Institute (INCOR-HCFMUSP), University of Sa~o Paulo Medical School, Av. Doutor Ene'as Carvalho Aguiar 44, 05403.000 Sa~o Paulo, Brazil Received 24 March 2006; accepted after revision 4 November 2006

22. Parameters characterizing implantable defibrillator output: a proposal Werner Irnich Europace Advance Access originally published online on June 5, 2007

23. Four years experience of a nurse-led elective cardioversion service within a district general hospital setting. Rhidian J. Shelton1*, Alan Allinson1, Tracey Johnson1, Charles Smales 2, and Gerald C. Kaye1Department of Cardiology, Castle Hill Hospital, Cottingham, Kingston-upon-Hull HU16 5JQ, UK; and 2Department of Anaesthetics, Castle Hill Hospital, Kingston-upon-Hull, UK

24. A simple point score system for predicting the efficacy of external rectilinear biphasic cardioversion for persistent atrial fibrillation. Sebastian Stec*, Aleksander Gorecki, Beata Zaborska, and Piotr Kulakowski Received 6 May 2005; accepted after revision 15 January 2006; online publish-ahead-of-print 16 March 2006

25. ACC/AHA/ESC 2006 guidelines for the management of patients with atrial fibrillation: www.circ.ahajournals.org/cgi/content/full/95/7/932

26. Defibrillation Efficacy of Commercially Available Biphasic Impulses in Humans: Importance of Negative-Phase Peak Voltage. Gery Tomassoni, MD; Keith Newby, MD; Sanjay Deshpande, MD; Kathi Axtell, RN; Jasbir Sra, MD; Masood Akhtar, MD; Andrea Natale, MD www.circ.ahajournals.org/cgi/content/full/95/7/1822#F1

27. "Recomendaciones 2005 del European Resuscitation Council sobre Reanimación Cardiopulmonar. Sección 3. Tratamientos eléctricos: desfibriladores externos automáticos, desfibrilación, cardioversión y marcapasos." (Recommendations 2005 of the European Resuscitation Council on Cardiopulmonary Resuscitation. Section 3. Electric treatments: automatic external defibrillators, defibrillation, cardioversion and pacemakers) Charles D. Deakin, Jerry P. Nolan

28. Possibilities for predictive measurement of the transthoracic impedance in defibrillation
Auteur(s) / Author(s) Rasteva V. (1); A1 Hatib F. (1); Trendafilova E. (2); Daskalov 1. (1); Affiliation(s) du ou des auteurs / Author(s) Affiliation(s) Journal of medical engineering & technology ISSN 0309-1902 2001, vol. 25, no5, pp. 195-200 (15 ref.)

29. Transthoracic electrical impedance during external defibrillation: comparison of measured and modelled waveforms F Al Hatib et al. 2000 Physio. Meas. 21 145-153

30. "Umbral de impedancia transtorácica para la detección del paciente en desfibrilación" (Transthoracic impedance threshold to detect the patient in defibrillation) Colorado, Osmani Portela, Alejandro Ernesto Folgueras, José University of Science and Technology, Volume 11, N° 44, September 2007. pp 129-134

31. IEC60601-2-4 IEC 60601-2-4 Medical Electrical Equipment Part 2-4: Particular Requirements for the Safety of Cardiac Defibrillators

32. Wikipedia: http://es.wikipedia.org.desfibrilacion

33. Cellular excitation with high-frequency chopped defibrillator waveforms Jones, J.L.; Sweeney, R.J.; Milne, K.B. Engineering in Medicine and Biology Society, 1994. Engineering Advances: New Opportunities for Biomedical Engineers. Proceedings of the 16th Annual International Conference of the IEEE Volume , Issue , 3-6 Nov 1994 Page (s):17 - 18 vol.1 http://ieeexplore.ieee.org/Xphore/login.jsp?url=jel5/3230/9197/0012168.pdf/temp =x

34. External defibrillators and Emergency and External Pacemakers Charbonier, IEEE Proceedings Vol 84 n°3 p487-499 http;//ieeexcplore.ieee.org.xpl/freeabs all.jsp?arnumber=486750

35. US patent n° 006647290
Charge based defibrillation method and apparatus

36. PCT/FR01/01419 patent
Modulated Defibrillation elementary pulse train

37. US patent n° 00 6671546-B2
Impulses or a series of impulses for defibrillation and device to generate them

38. PCT/EP02/14723
Defibrillation signal, method and device for adjusting defibrillation energy relative to patient's transthoracic resistance

39. US patent n$^n$ US6208896
Method and apparatus for providing variable defibrillation waveforms using switch-mode amplification

40. US Patent n°007283871
Self adjusting optimal waveforms

41. US patent n°007398122-B1
Self adjusting optimal waveforms

42. US Patent n°006067158
Defibrillator having a secure discharging circuit comprising an H-bridge

43. PTC/US98/07669
Defibrillator method and apparatus

44. US Patent n°005824017
H-Bridge circuit for generating a High-energy biphasic waveform in an external defibrillator

45. US7200434-B2
Control of arbitrary waveforms for constant delivered energy

46. PCT/US01/28899
Method and apparatus for delivering a biphasic defibrillation pulse with variable energy

47. PCT/US9706003
External defibrillator having low capacitance and small time constant

48. US Patent n° 007194303
H-Bridge with Sensing circuit

49. PTC/US2007/008686
Automated Defibrillator

50. PTC-US2005-042620
Automated External Defibrillator (AED) with discrete sensing pulse for use in configuring a therapeutic biphasic wave

51. "Un método de ajuste de la energia para un desfibrilador bifásico basado en la impedancia transtorácica" (An adjustment method of energy for a biphasic defibrillator based in the transthoracic impedance)
A. Portela, J. Folgueras
"Memorias del V Congreso de la Sociedad Cubana de Bioingeniería" (V Congress of the Bioengineering Cuban Society Report)

52. Biphasic defibrillation pulses: their influence on the survival rate in prehospital resuscitation
Albert Cansell, PhD

53. Transmembrane potential changes caused by monophasic and biphasic shocks Xiaohong Zhou; William Smith;

Robert K. Justice; James L. Wayland and Raymond E. Ideker. Am J Physiol Heart Circ Physiol, Nov 1998; 275: H1798 - H1807

54. Transmembrane potential changes caused by shocks in guinea pig papillary muscle X. Zhou, W. M. Smith, D. L. Rollins, and R. E. Ideker Am J Physiol Heart Circ Physiol, Dec 1996; 271: H2536 - H2546.

55. Dose-dependent reduction of cardiac transmembrane potential by high-intensity electrical shocks Michel Neunlist and Leslie Tung Am J Physiol Heart Circ Physiol, Dec 1997; 273: H2817 - H2825

56. Nonlinear changes of transmembrane potential caused by defibrillation shocks in strands of cultured myocytes Vladimir G. Fast, Stephan Rohr, and Raymond E. Ideker Am J Physiol Heart Circ Physiol, Jun 2004; 286: H2393 - H2400.

57. Optimal Duration for Defibrillation Shocks
http://www.zoll.com/page.aspx/id=306

58. New Control for Patient Impedance
http://www.zoll.com/page.aspx?id=299

59. Experimental verification of theoretical predictions concerning the Optimum Defibrillation Waveform Robert A. Malkin*, Senior Member, IEEE, Stephanie R. Jackson, Jennifer Nguyen, Zhao Yang, and Dongxu Guan, Member, IEEE IEEE Transactions on Biomedical Engineering, Vol. 53, No. 8, August 2006

60. The Effect of Phasic Duration on the Excitation Threshold of Biphasic Waveforms: a Computer Model Study Weilun Quan and Steven J Evans Computers in Cardiology 1995 Volume , Issue , 10-13 Sep 1995 Page(s):341 - 343

61. Effect of Biphasic Shock Duration on Defibrillation Threshold With Different Electrode Configurations and Phase 2 Capacitances. Patrick N. Schauerte, MD; Kathrin Ziegert, MD; Matthias Waldmann, BSE; Friedrich A. Schöndube, MD; Frank Birkenhauer, BSE; Karl Mischke, BSE; Marius Grossmann, MD; Peter Hanrath, MD; Christoph Stellbrink, MD Circulation. 1999;99:1516-1522

62. Strength-duration relationship for biphasic, defibrillation in dogs Lang, D.J.; Swanson, D.K.; Bach, S.M., Jr.; Shapland, J.E. Engineering in Medicine and Biology Society, 1989. Images of the Twenty-First Century., Proceedings of the Annual International Conference of the IEEE Engineering in Volume , Issue , 9-12 Nov 1989 Page(s):80 - 81 vol.1

63. Defibrillation with low-energy biphasic waveform reduces the severity of post-resuscitation myocardial dysfunction after prolonged cardiac arrest. Reddy RK, Gleva MJ, Gliner BE et al.. Journal of Critical Care Medicine. 1999; 27:A43

64. Is 360 joules necessary for biphasic transthoracic cardioversion of atrial fibrillation? Koul A, Rashba EJ, Shorofsky SR et al. PACE. 2002 24:688

65. Comparison of three Biphasic Defibrillation Waveforms: Gurvich Waveform is more efficient F. Qu, V.P. Nikolski, B:R: Wollenzier, I:R: Efimov. Proceedings of the Second Joint EMBSBMES Conference Huston Tx. October 23-25, 2002

66. The effect of temporal separation of phases on biphasic waveform defibrillation efficacy Randolph AS. Cooper, Steven T. Wallenius, William M. Smith, and Raymond E. Ideker Electrocardiography 15.18-6.

67. Prediction of Transthoracic Impedance to Defibrillating Current in External Defibrillation

68. Thoracic impedance of human subjects J.W. Machin Medical&Biological Engineering&Computing. March 1978

69. Changes in chest electrode impedance. The American Journal of Emergency Medicine, Volume 18, Issue 4 , Pages 381 - 384 F. Lateef

70. Relationship between canine transthoracic impedance and defibrillation threshold. Evidence for current-based defibrillation. B B Lerman, H R Halperin, J E Tsitlik, K Brin, C W Clark, and O C Deale

71. The prediction of the impedance of the thorax to defibrillating current Med. Instruí 1976 May-Jun;10(3):159-62. Geddes LA, Tacker WA Jr, Schoenlein W, Minion M, Grubbs S, Wilcox P.

72. Mechanisms responsible for decline in transthoracic impedance after DC shocks. S. J. Sirna, R. A. Kieso, K. J. Fox-Eastham, J. Seabold, F. Charbonnier and R. E. Kerber. Am J Physiol Heart Circ Physiol 257: H1180-H1183, 1989

73. Automatic adjustment of chopping-modulated defibrillation pulses to patient transthoracic resistance. V. Krasteva; A. Cansell; I. Daskalov. Journal of Medical Engineering & Technology, Volume 27 , Issue 1January 2003 , pages 11 - 18

74. Transthoracic impedance study with large self-adhesive electrodes in two conventional positions for defibrillation. Krasteva, Vessela; Matveev, Mikhail; Mudrov, Nikolay; Prokopova, Rada. Physiological Measurement, Volume 27, Issue 10, pp. 1009-1022 (2006).

75. Initial experience with a microprocessor controlled current based defibrillator. G W Dalzell, S R Cunningham, J Anderson, A A Adgey. British Heart Journal 1989;61:502-505; doi: 10.1136/hrt.61.6.502

76. The Class D Amplifier. Introduction to Electroacoustics and Audio Amplifier Design, Second Edition - Revised Printing, by W. Marshall Leach, Jr., published by Kendall/Hunt, © 2001.

77. Experimental Verification of Theoretical Predictions Concerning the Optimum Defibrillation Waveform Robert A. Malkin*, Senior Member, IEEE, Stephanie R. Jackson, Jennifer Nguyen, Zhao Yang, and Dongxu Guan IEEE Transactions on Biomedical Engineering, Vol. 53, No. 8, August 2006

78. Strength-duration relationship for biphasic defibrillation in dogs Douglas J. Lang, David K. Swanson, Stanley M. Bach, Jr., and J. Edward Shapland IEEE Engineering in Medicine & Biology Society 11th Annual International Conference

79. The effect of temporal separation of phases on biphasic waveform defibrillation efficacy.
Randolph AS. Cooper, Steven T. Wallenius, William M. Smith, and Raymond E. Ideker Annual International Conference of the IEEE Engineering in Medicine and Biology Society, Vol. 13, No. 2. 1991

80. The Effect of Phasic Duration on the Excitation Threshold of Biphasic Waveforms: a Computer Model Study Weilun Quan and Steven J Evans Computers in Cardiology 1995

81. Preliminary Results on the Performance of a Biphasic Cuban Defibrillator in Swine J. Folgueras, A. E. Portela A. Milands , 0. Colorado, L. A. Guevara, F. J. Tornés, D. Druyet, 0. Creagh, E. Domínguez Proceedings of the 25th Annual International Conference of the IEEE EMBS Cancun, Mexico September 17-21,2003

82. Improving Defibrillation With Low Energy Biphasic Waveforms. Zoll Corp. http://rces.nb.ca/seminar/old seminars/ 2004/Presentations/Energycris.pdf

83. Transthoracic Electrical Impedance During External Defibrillation. VT Krasteva

84. Sistema de Alimentación Ininterrumpido de 1kW" (System of Uninterrupted Power Supply of 1kW). Gustavo Ernesto Carranza, Revista Telegráfica Electrónica n° 869 November 1985.

## Claims

1. A control system to generate defibrillation waves of automatically compensated charge without measurement of the patient impedance, wherein the charge delivered to each patient is independent from the value of the impedance, so as both the charges in a straight and in an inverse direction result exclusively a function of the selected dose, either energy or charge, and constant for different values of patient impedance.

2. A control system according to claim 1, wherein it delivers the charge in pulses and this charge is proportional to the remaining instant charge in the storage capacitor in the end of each pulse or sample cycle.

3. A control system according to claim 1, wherein it delivers to the patient a fixed amount of charge in the straight direction and other in the inverse direction, lower and in an adjustable proportion according to the physiological requirements, in order to restore to zero the membrane potentials of the myocardium cells.

4. A control system according to claim 1, wherein the necessary voltage in the energy capacitor is independent from the patient impedance within the established range.

5. A control system according to claim 1, wherein it delivers the charge to the patient through pulses of a controlled wide of a much higher frequency than the inverse of the total time of energy delivery.

6. A control system according to claim 1, wherein it uses an "H-bridge" that initiates each cycle connecting to the patient with the suitable direction measuring the delivered energy and integrating the same on a capacitor until the charge is the desired one.

7. A control system according to claim 1, wherein it compares the charge delivered to the patient (integral to the current) with a fraction of the tension present in the capacitor and annuls the current in the instant that the integral of the current reaches the value of said fraction.

8. A control system according to claim 1, wherein it replaces the comparison tension (fraction of the present in the condenser) by a controlled tension, allowing to program pulse by pulse the amount of charge delivered, meanwhile the wide of the pulse resulting does not reach the sample time.

9. A control system according to claims 1 and 8, wherein it allows the complete configuration, within the limit established in claim 8, of the charge/time distribution during the discharge.

FIG. 1

FIGURE 2

## PATIENT CURRENT

## NET PATIENT CHARGE

## FIGURE 3

**FIGURE 4**

EP 2 229 978 A1

FIGURE 5

19

Europäisches
Patentamt

European
Patent Office

Office européen
des brevets

# EUROPEAN SEARCH REPORT

Application Number

EP 10 15 4339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 735 879 A (GLINER BRADFORD E [US] ET AL) 7 April 1998 (1998-04-07) | 1-4,8,9 | INV. A61N1/39 |
| Y | * column 2, line 31 - column 5, line 64; figures 6-8 * | 5-7 | |
| Y | US 6 493 580 B1 (CANSELL ALBERT [FR] ET AL) 10 December 2002 (2002-12-10) * column 2, line 36 - column 5, line 6; figures 1A-1B * | 5 | |
| Y,D | US 6 647 290 B2 (WUTHRICH SCOTT A [US]) 11 November 2003 (2003-11-11) * column 14, line 46 - column 21, line 22; figures 4,8 * | 6,7 | |
| A | US 6 208 896 B1 (MULHAUSER DANIEL F [US]) 27 March 2001 (2001-03-27) * the whole document * | 1-9 | |
| A | WO 2006/067158 A1 (SCHILLER MEDICAL SAS [FR]; FOELLER CLEMENT [FR]; SCHILLER ALFRED [CH];) 29 June 2006 (2006-06-29) * the whole document * | 1-9 | TECHNICAL FIELDS SEARCHED (IPC) A61N |

The present search report has been drawn up for all claims

| Place of search | Date of completion of the search | Examiner |
|---|---|---|
| Munich | 15 July 2010 | Smit, Jos |

EPO FORM 1503 03.82 (P04C01)

## ANNEX TO THE EUROPEAN SEARCH REPORT
## ON EUROPEAN PATENT APPLICATION NO.

EP 10 15 4339

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2010

| Patent document cited in search report | | Publication date | Patent family member(s) | | Publication date |
|---|---|---|---|---|---|
| US 5735879 | A | 07-04-1998 | US | 5607454 A | 04-03-1997 |
| | | | US | 5601612 A | 11-02-1997 |
| | | | US | 5593427 A | 14-01-1997 |
| | | | US | 5749905 A | 12-05-1998 |
| US 6493580 | B1 | 10-12-2002 | AT | 346650 T | 15-12-2006 |
| | | | DE | 60032039 T2 | 05-07-2007 |
| | | | DK | 1023920 T3 | 10-04-2007 |
| | | | EP | 1023920 A1 | 02-08-2000 |
| | | | ES | 2277823 T3 | 01-08-2007 |
| | | | FR | 2788699 A1 | 28-07-2000 |
| US 6647290 | B2 | 11-11-2003 | DE | 60032448 T2 | 11-10-2007 |
| | | | EP | 1118353 A2 | 25-07-2001 |
| | | | JP | 4364443 B2 | 18-11-2009 |
| | | | JP | 2001218855 A | 14-08-2001 |
| | | | JP | 2009154008 A | 16-07-2009 |
| | | | US | 2003074025 A1 | 17-04-2003 |
| US 6208896 | B1 | 27-03-2001 | NONE | | |
| WO 2006067158 | A1 | 29-06-2006 | EP | 1830922 A1 | 12-09-2007 |
| | | | FR | 2879937 A1 | 30-06-2006 |
| | | | KR | 20070114116 A | 29-11-2007 |
| | | | RU | 2365389 C2 | 27-08-2009 |

EPO FORM P0459

For more details about this annex : see Official Journal of the European Patent Office, No. 12/82

## REFERENCES CITED IN THE DESCRIPTION

**Patent documents cited in the description**

- US 006647290 B
- FR 0101419 W
- US 006671546 B2
- EP 0214723 W
- US 6208896 B
- US 007283871 B
- US 007398122 B1
- US 006067158 A

- US 9807669 PCT
- US 005824017 A
- US 7200434 B2
- US 0128899 W
- US 9706003 W
- US 007194303 B
- US 2007008686 PCT
- US 2005042620 PCT

**Non-patent literature cited in the description**

- External Defibrillators. **Tacker, W. A.** The Biomedical Engineering Handbook. Boca Raton: CRC Press LLC, 2000
- **Marin I. ; Hernández Madrid A. ; Gómez Bueno M. ; Escobar Cervantes C. ; Moro Serrano C.** Cardioversión Bifásica vs Monofásica en una serie consecutiva. ¿Qué aportan de mejora los nuevos desfibriladores bifásicos frente a los convencionales?. *Biphasic vs Monophasic Cardioversion in a consecutive series. What is the contribution of the new biphasic defibrillators compared to the conventional ones?*
- **Derek J. Dosdall ; Raymond E. Ideker.** *Heart Rhythm.,* March 2007, vol. 4 (3), 51-856
- **Tim Risiusl ; Tjark F ; Schwemer ; Muhammet Ali Aydin ; Ralf Köster ; Hanno U. Klemm ; Boris Lutomsky ; Thomas Meinertz ; Rodolfo Ventura ; Stephan Willems.** Biphasic versus Monophasic Shock for External Cardioversion of Atrial Flutter. *A Prospective, Randomized Trial Kai Mortensen1*
- **Osnat T. Gurevitz ; MDa, Naser M. Animash ; MDa, Joseph F. Malouf ; MDa, Krishnaswamy Chandrasekaran ; MDa, Ana Gabriela Rosales ; MSb, Karla V. Ballman ; PhDb, Stephen C. Hammill ; MDa, Roger D. White ; MDc, Bernard J. Gersh ; MB, ChBa, Paul A. Friedman.** *Comparative efficacy of monophasic and biphasic waveforms for transthoracic cardioversion of atrial fibrillation and atrial flutter,* 20 January 2004
- **Richard O. Cummins ; Mary Fran Hazinski ; Richard E. Kerber ; Peter Kudenchuk ; Lance Becker ; Graham Nichol ; Barbara Malanga ; Tom P. Aufderheide ; Edward M. Stapleton ; Karl Kern.** *Circulation,* 1998, vol. 97, 1654-1557

- *Indian Pacing and Electrophysiology Journal,* 2005, vol. 5 (4), ISSN 0972-6292, 289-295
- **JF Swartz ; JL Jones ; RE Jones ; R Fletcher.** *Circ. Res.,* 1991, vol. 68, 438-449
- **Elaine Amato-Vealey, RN ; Patricia A. Colonies, RN.** *CCRN, Med Nursing2005,* August 2005, vol. 35 (8), 6-11
- **Berg, Marc D. MD ; Banville, Isabelle L. PhD ; Chapman, Fred W. PhD ; Waker, Robert G. BS ; Gaballa, Mohammed A. PhD ; Hilwig, Ronald W. PhD, DVM ; Samson, Ricardo A. MD ; Kern, Karl B. MD ; Berg, Robert A. MD.** *Laboratory Investigations Pediatric Critical Care Medicine,* July 2006, vol. 9 (4), 429-434
- **Rasteva V. (1) ; A1 Hatib F. (1) ; Trendafilova E. (2) ; Daskalov 1. (1).** *Journal of medical engineering & technology,* 2001, vol. 25 (5), ISSN 0309-1902, 195-200
- **F Al Hatib et al.** *Physio. Meas.,* 2000, vol. 21, 145-153
- Umbral de impedancia transtorácica para la detección del paciente en desfibrilación. **Osmani Portela ; Alejandro Ernesto Folgueras.** Transthoracic impedance threshold to detect the patient in defibrillation. José University of Science and Technology, September 2007, vol. 11, 129-134
- **Jones, J.L. ; Sweeney, R.J. ; Milne, K.B.** Cellular excitation with high-frequency chopped defibrillator waveforms. *Engineering in Medicine and Biology Society, 1994. Engineering Advances: New Opportunities for Biomedical Engineers. Proceedings of the 16th Annual International Conference of the IEEE,* 03 November 1994, vol. 1, 17-18
- External defibrillators and Emergency and External Pacemakers Charbonier. *IEEE Proceedings,* vol. 84 (3), 487-499

- **Xiaohong Zhou ; William Smith ; Robert K. Justice ; James L. Wayland ; Raymond E. Ideker.** Transmembrane potential changes caused by monophasic and biphasic shocks. *Am J Physiol Heart Circ Physiol,* November 1998, vol. 275, H1798-H1807
- **X. Zhou ; W. M. Smith ; D. L. Rollins ; R. E. Ideker.** Transmembrane potential changes caused by shocks in guinea pig papillary muscle. *Am J Physiol Heart Circ Physiol,* December 1996, vol. 271, H2536-H2546
- **Michel Neunlist ; Leslie Tung.** Dose-dependent reduction of cardiac transmembrane potential by high-intensity electrical shocks. *Am J Physiol Heart Circ Physiol,* December 1997, vol. 273, H2817-H2825
- **Vladimir G. Fast ; Stephan Rohr ; Raymond E. Ideker.** Nonlinear changes of transmembrane potential caused by defibrillation shocks in strands of cultured myocytes. *Am J Physiol Heart Circ Physiol,* June 2004, vol. 286, H2393-H2400
- **Robert A. Malkin ; Senior Member ; Stephanie R. Jackson ; Jennifer Nguyen ; Zhao Yang ; Dongxu Guan.** Experimental verification of theoretical predictions concerning the Optimum Defibrillation Waveform. *Member, IEEE IEEE Transactions on Biomedical Engineering,* August 2006, vol. 53 (8
- **Weilun Quan ; Steven J Evans.** The Effect of Phasic Duration on the Excitation Threshold of Biphasic Waveforms: a Computer Model Study. *Computers in Cardiology,* 10 September 1995, 341-343
- **Patrick N. Schauerte, MD ; Kathrin Ziegert, MD ; Matthias Waldmann, BSE ; Friedrich A. Schöndube, MD ; Frank Birkenhauer, BSE ; Karl Mischke, BSE ; Marius Grossmann, MD ; Peter Hanrath, MD ; Christoph Stellbrink, MD.** Effect of Biphasic Shock Duration on Defibrillation Threshold With Different Electrode Configurations and Phase 2 Capacitances. *Circulation,* 1999, vol. 99, 1516-1522
- **Lang, D.J. ; Swanson, D.K. ; Bach, S.M., Jr. ; Shapland, J.E.** Strength-duration relationship for biphasic, defibrillation in dogs. *Engineering in Medicine and Biology Society, 1989. Images of the Twenty-First Century., Proceedings of the Annual International Conference of the IEEE Engineering,* 09 November 1989, vol. 1, 80-81
- **Reddy RK ; Gleva MJ ; Gliner BE et al.** Defibrillation with low-energy biphasic waveform reduces the severity of post-resuscitation myocardial dysfunction after prolonged cardiac arrest. *Journal of Critical Care Medicine,* 1999, vol. 27, A43
- **F. Qu ; V.P. Nikolski ; B:R: Wollenzier ; I:R: Efimov.** Comparison of three Biphasic Defibrillation Waveforms: Gurvich Waveform is more efficient. *Proceedings of the Second Joint EMBSBMES Conference Huston Tx,* 23 October 2002
- **Randolph AS. Cooper ; Steven T. Wallenius ; William M. Smith ; Raymond E. Ideker.** The effect of temporal separation of phases on biphasic waveform defibrillation efficacy. *Electrocardiography,* vol. 15, 18-6
- **J.W. Machin.** Thoracic impedance of human subjects. *Medical&Biological Engineering&Computing,* March 1978
- **F. Lateef.** Changes in chest electrode impedance. *The American Journal of Emergency Medicine,* vol. 18 (4), 381-384
- **B B Lerman ; H R Halperin ; J E Tsitlik ; K Brin ; C W Clark ; O C Deale.** *Relationship between canine transthoracic impedance and defibrillation threshold. Evidence for current-based defibrillation*
- **Geddes LA ; Tacker WA Jr ; Schoenlein W ; Minion M ; Grubbs S ; Wilcox P.** The prediction of the impedance of the thorax to defibrillating current. *Med. Instruí,* May 1976, vol. 10 (3), 159-62
- **S. J. Sirna ; R. A. Kieso ; K. J. Fox-Eastham ; J. Seabold ; F. Charbonnier ; R. E. Kerber.** Mechanisms responsible for decline in transthoracic impedance after DC shocks. *Am J Physiol Heart Circ Physiol,* 1989, vol. 257, H1180-H1183
- **V. Krasteva ; A. Cansell ; I. Daskalov.** Automatic adjustment of chopping-modulated defibrillation pulses to patient transthoracic resistance. *Journal of Medical Engineering & Technology,* January 2003, vol. 27 (1), 11-18
- **Krasteva, Vessela ; Matveev, Mikhail ; Mudrov, Nikolay ; Prokopova, Rada.** Transthoracic impedance study with large self-adhesive electrodes in two conventional positions for defibrillation. *Physiological Measurement,* 2006, vol. 27 (10), 1009-1022
- **G W Dalzell ; S R Cunningham ; J Anderson ; A A Adgey.** *British Heart Journal,* 1989, vol. 61, 502-505
- **W. Marshall Leach, Jr.** The Class D Amplifier. Introduction to Electroacoustics and Audio Amplifier Design. Kendall/Hunt, 2001
- **Robert A. Malkin ; Stephanie R. Jackson ; Jennifer Nguyen ; Zhao Yang ; Dongxu Guan.** Experimental Verification of Theoretical Predictions Concerning the Optimum Defibrillation Waveform. *IEEE Transactions on Biomedical Engineering,* August 2006, vol. 53 (8
- **Douglas J. Lang ; David K. Swanson ; Stanley M. Bach, Jr. ; J. Edward Shapland.** Strength-duration relationship for biphasic defibrillation in dogs. *IEEE Engineering in Medicine & Biology Society 11th Annual International Conference*
- **Randolph AS. Cooper ; Steven T. Wallenius ; William M. Smith ; Raymond E.** *Ideker Annual International Conference of the IEEE Engineering in Medicine and Biology Society,* 1991, vol. 13 (2

- The Effect of Phasic Duration on the Excitation Threshold of Biphasic Waveforms: a Computer Model Study Weilun Quan and Steven J Evans Computers. *Cardiology,* 1995

- **J. Folgueras ; A. E. Portela ; A. Milands ; 0. Colorado ; L. A. Guevara ; F. J. Tornés ; D. Druyet ; 0. Creagh ; E. Domínguez.** *Proceedings of the 25th Annual International Conference of the IEEE EMBS Cancun,* 17 September 2003
- **Gustavo Ernesto Carranza.** *Revista Telegráfica Electrónica,* November 1985